# EUROPEAN PATENT SPECIFICATION

(11) **EP 1 079 230 B1**
(45) Date of publication and mention of the grant of the patent: **10.08.2005**
(21) Application number: 99913652.6
(22) Date of filing: 13.04.1999
(51) Int. Cl.: G01N 33/556, G01N 33/48, G01N 33/92, G01N 33/566

(54) **METHOD FOR SCREENING SUBSTANCE PROMOTING OLIGOMERIZATION OF RECEPTOR PROTEIN MOLECULES**
VERFAHREN ZUM SCREENEN EINER DIE OLIGOMERISIERUNG VON REZEPTORPROTEINMOLEKÜLEN FÖRDERNDEN SUBSTANZ
PROCEDE DE RECHERCHE D'UNE SUBSTANCE FAVORISANT L'OLIGOMERISATION DE MOLECULES DE PROTEINES RECEPTRICES

(30) Priority: 14.04.1998 JP 10232598
(43) Date of publication of application: 28.02.2001
(73) Proprietor: CHUGAI SEIYAKU KABUSHIKI KAISHA, Tokyo, 115-8543 (JP)
(72) Inventor: HIGUCHI, M., Chugai Seiyaku Kabushiki Kaisha, Shizuoka 412-8543 (JP); SHIMONAKA, Y., Chugai Seiyaku Kabushiki Kaisha, Shizuoka 412-85 43 (JP); ESAKI, Keiko Chugai Seiyaku Kabushiki Kaisha, Shizuoka 412-8543 (JP)
(74) Representative: Harrison, David Christopher
(86) International application number: PCT/JP1999/001965
(87) International publication number: WO 1999/053313

(56) References cited:
- JP-A- 6 167 494
- JP-A- 6 507 715
- JP-A- 62 047 555
- WATOWICH S S ET AL: "ACTIVATION AND INHIBITION OF ERYTHROPOIETIN RECEPTOR FUNCTION: ROLE OF RECEPTOR DIMERIZATION" MOLECULAR AND CELLULAR BIOLOGY, AMERICAN SOCIETY FOR MICROBIOLOGY, WASHINGTON, US, vol. 14, no. 6, 1994, pages 3535-3554, XP001008074 ISSN: 0270-7306
- ELLIOTT S ET AL: "ACTIVATION OF THE ERYTHROPOIETIN (EPO) RECEPTOR BY BIVALENT ANTI-EPO RECEPTOR ANTIBODIES" JOURNAL OF BIOLOGICAL CHEMISTRY, AMERICAN SOCIETY OF BIOLOGICAL CHEMISTS, BALTIMORE, MD, US, vol. 271, no. 40, 4 October 1996 (1996-10-04), pages 24691-24697, XP002044305 ISSN: 0021-9258
- MIURA O ET AL: "DIMER- AND OLIGOMERIZATION OF THE ERYTHROPOIETIN RECEPTOR BY DISULFIDE BOND FORMATION AND SIGNIFICANCE OF THE REGION NEAR THE WSXWS MOTIF IN INTRACELLULAR TRANSPORT" ARCHIVES OF BIOCHEMISTRY AND BIOPHYSICS, NEW YORK, US, US, vol. 306, no. 1, 1993, pages 200-208, XP001008073 ISSN: 0003-9861
- WATOWICH S S ET AL: "OLIGOMERIZATION AND CONSTITUTIVE ACTIVATION OF THE ERYTHROPOIETIN RECEPTOR" JOURNAL OF CELLULAR BIOCHEMISTRY. SUPPLEMENT, A.R. LISS, NEW YORK, NY, US, no. 16, PART C, 1992, page 104 XP001008072 ISSN: 0733-1959

### SEQUENCE LISTING

<110> Chugai Seiyaku Kabushiki Kaisha
<120> A method for screening substances capable of promoting oligomerization of receptor protein molecules
<130> DCH/FP5888359
<140> EP 99913652.6
   <141> 1999-04-13
<150> PCT/JP99/01965
   <151> 1999-04-13
<150> JP 10-102325
   <151> 1998-04-14
<160> 9
<170> PatentIn Ver. 2.0
<210> 1
   <211> 20
   <212> DNA
   <213> Artificial Sequence
<220>
   <223> Description of Artificial Sequence:synthetic DNA
<400> 1
   gcagctgctg acccagctgt 20
<210> 2
   <211> 20
   <212> DNA
   <213> Artificial Sequence
<220>
   <223> Description of Artificial Sequence:synthetic DNA
<400> 2
   aagtcttgag tctgcactgg 20
<210> 3
   <211> 29
   <212> DNA
   <213> Artificial Sequence
<220>
   <223> Description of Artificial Sequence:synthetic DNA
<400> 3
   tttgaattct gtatcatgga ccacctcgg 29
<210> 4
   <211> 29
   <212> DNA
   <213> Artificial Sequence
<220>
   <223> Description of Artificial Sequence:synthetic DNA
<400> 4
   tttaagcttg atccctgatc atctgcagc 29
<210> 5
   <211> 35
   <212> DNA
   <213> Artificial Sequence
<220>
   <223> Description of Artificial Sequence:synthetic DNA
<400> 5
   ttttaagaat tccaccatgg accacctcgg ggcgt 35
<210> 6
   <211> 54
   <212> DNA
   <213> Artificial Sequence
<220>
   <223> Description of Artificial Sequence:synthetic DNA
<400> 6
   gggatcctta tttatcgtca tcgtctttgt agtcgctagg cgtcagcagc gaca 54
<210> 7
   <211> 26
   <212> DNA
   <213> Artificial Sequence
<220>
   <223> Description of Artificial Sequence:synthetic DNA
<400> 7
   tgaattcagt gtgtgctgag caacct 26
<210> 8
   <211> 24
   <212> DNA
   <213> Artificial Sequence
<220>
   <223> Description of Artificial Sequence:synthetic DNA
<400> 8
   gggatccgct ttgctctcga actt 24
<210> 9
   <211> 42
   <212> PRT
   <213> Homo sapiens
<220>
   <221> DISULFID
   <222> (6)..(15)
<220>
   <221> DISULFID
   <222> (28)..(37)
<220>
   <221> MOD_RES
   <222> (22)
   <223> bAla
<400> 9
   Gly Gly Thr Tyr Ser Cys His Phe Gly Pro Leu Thr Trp Val Cys Lys
   1 5 10 15
   Pro Gln Gly Gly Lys Xaa Gly Gly Gln Pro Lys Cys Val Trp Thr Leu
   20 25 30
   Pro Gly Phe His Cys Ser Tyr Thr Gly Gly
   35 40

## Description

### TECHNICAL FIELD

The present invention relates to a method for screening substances that promote oligomerization of receptor protein molecules. More specifically, it relates to a method for screening physiologically active substances, which uses the oligomerization of receptor protein molecules as an indicator.

### BACKGROUND ART

Physiologically active substances, such as erythropoietin and growth hormone, contribute to transduction of differentiative and proliferative signals through their binding to specific receptors expressed on the target cell membrane. In 1990, Yoshimura et al. reported that an EPO receptor carrying a mutation of Arg to Cys at position 129 in its extracellular domain induces transduction of a proliferative signal even in the absence of EPO, suggesting that it is important for signal transduction to multimerize the extracellular domains of EPO receptors via disulfide linkages (Nature, 348: 647-649, 1990). Further, X-ray structure analysis of the growth hormone-growth hormone receptor binding indicated that two growth hormone receptors bind to one growth hormone molecule (Journal of Molecular Biology, 222(4):865-868, 1991). The receptor multimerization is thus more likely to contribute to the first step of the signal transduction pathway induced by physiologically active substances.

In 1996, an EPO receptor-binding peptide having a core structure of 14 amino acids YXCXXGPXTWXCXP (X may be any amino acid) was reported to possibly mimic EPO activity (Science, 273:458-463, 1996). Crystal structure analysis of this peptide and the EPO receptor complex indicated that peptide dimer promotes EPO receptor dimerization (Science, 273:464-471, 1996).

Furthermore, an antibody against an extracellular domain of the EPO receptor has been found to possibly mimic EPO activity. Monovalent (Fab) fragments of this antibody can bind to the EPO receptor, but not induce signal transduction, suggesting that EPO receptor dimerization by binding divalent antibody is required for its function (J. Biol. Chem., 271(40):24691-24697, 1996).

Such a signal transduction pathway triggered by receptor multimerization is observed not only in EPO and growth hormone, but also in other physiologically active substances including interferons and granulocyte colony-stimulating factor (G-CSF). With regard to thrombopoietin, a peptide has also been identified that may mimic thrombopoietin activity through receptor multimerization (Science, 276:1696-1699, 1997).

Cunningham et al. show a method for selecting agonists and antagonists by detecting ternary complex formation of growth hormone receptors and its ligands (Japanese Patent Application No. 5-500070). However, this method detects homoquenching of fluorescent probes using a spectrofluorometer, and hence is not suitable for large-scale screening of test substances.

The object of the present invention is to provide a screening method of substitutes for physiologically active substances, which uses receptor multimerization as an indicator.

### DISCLOSURE OF THE INVENTION

We have established a screening system permitting a convenient determination of whether receptor protein molecules or peptide fragments thereof are oligomerized by contact with test substance or not, and have thereby completed the invention.

The present invention provides a method for screening substances that promote oligomerization of receptor protein molecules or peptide fragments thereof, which comprises determining whether the receptor protein molecules or peptide fragments thereof are oligomerized when contacted with test substances. The test substance may be contacted with the receptor protein molecules or peptide fragments thereof in a cell-free environment.

In the above method, it is possible to measure signals generated by the oligomerization of the receptor protein molecules or peptide fragments thereof to determine whether the receptor protein molecules or peptide fragments thereof are oligomerized. The signals may be selected from the group consisting of scintillation signals, chemiluminescence signals, fluorescence signals, absorption signals, ionizing radiation signals, nuclear magnetic resonance signals, and surface plasmon resonance signals. Such signals may be measured by SPA method, RIA method, surface plasmon resonance, nuclear magnetic resonance, enzyme-linked immunosorbent assay, gel filtration chromatography and the like. At least one of the receptor protein molecules or peptide fragments thereof may be labeled. The label may be selected from the group consisting of radioisotope, fluorophore, enzyme, biotin, avidin, scintillant, and combinations thereof. To determine the presence of oligomerized molecules or fragments using SPA method, for example, at least one of the receptor protein molecules or peptide fragments thereof may be labeled with radioisotope, while at least one of the others may be attached to the surface of beads containing scintillant. In this case, a test substance can be added to an aqueous solution comprising the radiolabeled receptors or peptide fragments thereof as well as the receptors or peptide fragments thereof attached to the surface of scintillant-containing beads, and then the generated scintillation signals can be measured to determine whether the receptors or peptide fragments thereof are oligomerized. Those skilled in the art may properly determine the mixing ratio of the scintillant-containing beads (SPA beads) to the receptors to be attached to the beads according to the manufacturer's instructions.

The oligomers of the receptor protein molecules or peptide fragments thereof may be formed in a solution or on a solid surface. To determine the presence of the oligomers using nuclear magnetic resonance or gel filtration chromatography, in general, the oligomers are preferably formed in a solution. To determine the presence of the oligomers using SPA method, RIA method, surface plasmon resonance or enzyme-linked immunosorbent assay, in general, the oligomers are preferably formed on a solid surface.

The receptor may be a cytokine receptor selected from the group consisting of hematopoietic factor receptor, lymphokine receptor, growth factor receptor, and differentiation inhibitory factor receptor. More specifically, it may be selected from the group consisting of erythropoietin (EPO) receptor, thrombopoietin (TPO) receptor, granulocyte colony-stimulating factor (G-CSF) receptor, macrophage colony-stimulating factor (M-CSF) receptor, granulocyte-macrophage colony-stimulating factor (GM-CSF) receptor, tumor necrosis factor (TNF) receptor, interleukin-1 (IL-1) receptor, interleukin-2 (IL-2) receptor, interleukin-3 (IL-3) receptor, interleukin-4 (IL-4) receptor, interleukin-5 (IL-5) receptor, interleukin-6 (IL-6) receptor, interleukin-7 (IL-7) receptor, interleukin-9 (IL-9) receptor, interleukin-10 (IL-10) receptor, interleukin-11 (IL-11) receptor, interleukin-12 (IL-12) receptor, interleukin-13 (IL-13) receptor, interleukin-15 (IL-15) receptor, interferon-α (IFN-α) receptor, interferon-β (IFN-β) receptor, interferon-γ (IFN-γ) receptor, growth hormone (GH) receptor, insulin receptor, stem cell factor (SCF) receptor, vascular endothelial growth factor (VEGF) receptor, epidermal growth factor (EGF) receptor, nerve growth factor (NGF) receptor, fibroblast growth factor (FGF) receptor, platelet-derived growth factor (PDGF) receptor, transforming growth factor-β (TGF-β) receptor, leukemia inhibitory factor (LIF) receptor, ciliary neurotrophic factor (CNTF) receptor, oncostatin M (OSM) receptor, and Notch-like receptor.

The receptor protein molecule may be a cytokine receptor subunit. For example, each of IL-3 receptor, IL-5 receptor and GM-CSF receptor consists of α- and β-subunits. These receptors are known to share a common β-subunit. Each of IL-6 receptor, LIF receptor and IL-11 receptor consists of an α-subunit and gp130 subunit. These receptors are known to share a common gp130 subunit. Each of CNTF receptor and OSM receptor is a trimer of α-subunit, LIF receptor α-subunit and gp130 subunit. Also, each of IL-2 receptor, IL-4 receptor, IL-7 receptor, IL-9 receptor and IL-15 receptor consists of α-, β- and γ-subunits. These receptors are known to share a common γ-subunit.

The peptide fragment of the receptor protein molecule preferably comprises at least a ligand-binding site of the receptor. The receptor protein molecule has a ligand-binding site on its extracellular region or soluble region.

The oligomer that can be formed from the receptor protein molecules or peptide fragments thereof may be any homooligomer or heterooligomer including a dimer, trimer and tetramer. For example, erythropoietin receptor, thrombopoietin receptor, G-CSF receptor, SCF receptor and EGF receptor are known to form homodimers; IL-6 receptor, LIF receptor and IL-11 receptor are known to form heterodimers; and IL-2 receptor, CNTF receptor and OSM receptor are known to form heterotrimers.

A substance to be screened may be a novel substitute for a known physiologically active substance. The known physiologically active substance includes erythropoietin, interferon, G-CSF, growth hormone, thrombopoietin, GM-CSF and M-CSF.

In one embodiment of the above method, substances can be screened that promote dimerization of erythropoietin receptor protein molecules or peptide fragments thereof by adding a test substance to an aqueous solution comprising ¹²⁵I-labeled erythropoietin receptors or peptide fragments thereof as well as erythropoietin receptors or peptide fragments thereof attached to the surface of scintillant-containing yttrium silicate or polyvinyl toluene beads; and then measuring generated scintillation signals to determine whether the erythropoietin receptors or peptide fragments thereof are dimerized.

The present invention also provides a method for screening substances that inhibit the oligomerization of the receptor protein molecules or peptide fragments thereof, which comprises determining whether the oligomerization of the receptor protein molecules or peptide fragments thereof in the presence of an oligomerization-promoting substance is inhibited when contacted with a test substance. The oligomerization-promoting substance may have a physiological activity. The substance that inhibits the oligomerization of the receptor protein molecules or peptide fragments thereof may have the ability to inhibit the physiological activity of the oligomerization-promoting substance. For example, the oligomerization-promoting substance may be erythropoietin, and the receptor may be an erythropoietin receptor.

The present invention further provides a test kit for screening substances that inhibit the oligomerization of the receptor protein molecules or peptide fragments thereof, which comprises an oligomerization-promoting substance, the receptor protein molecules or peptide fragments thereof, and a buffer. For example, the oligomerization-promoting substance may be erythropoietin; the receptor protein molecule or peptide fragment thereof may be an erythropoietin receptor protein molecule or peptide fragment thereof; and the buffer may be phosphate buffered saline.

The present invention further provides the use of receptor protein molecules or peptide fragments thereof for determining whether a test substance promotes oligomerization of the receptor protein molecules or peptide fragments thereof.

The present invention will be further described below.

We first prepare receptor protein molecules and peptide fragments thereof. The receptor protein molecule or peptide fragment thereof may be in the form of a membrane fraction containing the receptor protein molecules or peptide fragments thereof, preferably may be isolated and purified from cells, more preferably may be produced recombinantly. For example, the receptor protein molecule or peptide fragment thereof may be prepared as follows.

When a membrane fraction is used, cells expressing the receptor protein molecules of interest may be solubilized in a buffer solution containing a surfactant such as Triton X-100, and then chromatographed using an affinity column on which ligands or anti-receptor antibodies have been immobilized in order to purify the receptor protein molecules.

When the receptor protein molecule has an identified ligand-binding site, a peptide fragment corresponding to this site may be synthesized and used in the present invention.

To produce the receptor protein molecule or peptide fragment thereof recombinantly, cDNA encoding the full-length receptor protein molecule or its extracellular region containing a ligand-biding site may be cloned using PCR method and cDNA library, and then integrated into an appropriate expression vector to express the gene of interest in a host cell such as *E. coli* or CHO cell. For the purpose of simple purification, a gene encoding a tag, such as MBP, GST or FLAG, may be ligated upstream or downstream of the gene encoding the protein of interest.

To detect the oligomer of the receptor protein molecules or peptide fragments thereof by RIA method or enzyme-linked immunosorbent assay, the receptor protein molecules or peptide fragments thereof have been previously labeled.

Next, to determine whether the receptor protein molecules or peptide fragments thereof are oligomerized, the molecules or fragments may be contacted with a test substance as follows.

In one embodiment of the present invention, we can measure the binding between receptors by scintillation technique. The scintillation technique can detect photon emission resulting from collisions between radioactive rays from some receptors labeled with radioisotopes and emission substances (i.e., scintillant) attached to the other receptors in oligomer complex. The resulting photon emission may be measured as a voltage pulse by means of a photomultiplier or a photodiode. The preferred emission substance includes crystals or powder of NaI, CsI and ZnS, or crystals of anthracene and naphthalene. Alternatively, scintillation proximity assay (SPA) technique developed by Amersham International (Bothworth N, and Towers P., 1989, Nature, 341, 167-168) can be used for this purpose. The SPA technique converts radiation energy from a radioisotope into light through absorption by scintillant-containing yttrium silicate or polyvinyl toluene beads (SPA beads) when the radioisotope binds to or is in close proximity to SPA beads. For example, some receptors are attached to SPA beads, while the other receptors are radiolabeled. Next, a test substance may be added to a solution comprising the radiolabeled receptors as well as the SPA beads-attached receptors, so as to detect the light resulting from the receptor oligomerization mediated by the test substance. Avidin and biotin can be used to attach the receptors to SPA beads. The radioisotope to be used includes ³H, ¹⁴C, ³²P, ⁴⁵Ca and ¹²⁵I. Alternatively, we can use a microtiter plate containing scintillant on its bottom surface in place of SPA beads. For example, unlabeled receptors are immobilized on the bottom surface of the plate, and radiolabeled receptors and test substance are added thereto.

Upon formation of the receptor oligomer mediated by the test substance, the radiolabeled receptors build up on the bottom surface of the plate and the scintillant contained in the bottom surface absorbs radiation energy from the radioisotope, thereby causing photon emission. Increased photon emission results in the detection of the receptor oligomerization. The method using SPA beads is convenient and suitable for large-scale screening of test substances for their activity because there is no need to separate unbound receptors from bound receptors before detection.

In another embodiment of the present invention, surface plasmon resonance sensor, called BIACORE (developed by and commercially available from Pharmacia Biotech) can be used to detect receptor oligomerization. BIACORE has a basic structure including a light source, a prism, a detector and a microchannel. Some receptors have been immobilized on a cassette-type sensor chip, and the other receptors are then injected onto the sensor chip. The injected receptors are supplied to the sensor chip at a constant rate of flow and then distributed over the immobilized receptors. Upon formation of the oligomer through binding of the immobilized receptors with the injected receptors, the injected receptors build up on the sensor chip, resulting in increased volume of proteins on the chip. This increased volume can be detected optically as a plasmon resonance signal. For example, a test substance may be injected simultaneously with the receptors to detect an increased intensity of plasmon resonance signal, allowing the detection of the test substance activity from an increased plasmon resonance signal induced by the presence of the test substance.

In a further embodiment of the present invention, a nuclear magnetic resonance (NMR) technique can be used to detect receptor oligomerization. The NMR technique uses a low-energy electromagnetic wave, and is preferred due to its high sensitivity to detect even small energy changes. For example, two types of receptors are dissolved in a solvent at around 1 mM, followed by the measurement of nuclear magnetic resonance signals. Subsequently, a test substance is added to the solution. Nuclear magnetic resonance signals can be measured again so as to detect the receptor oligomerization as a signal shift.

In a further embodiment of the present invention, enzyme-linked immunosorbent assay (ELIZA) can be used. For example, some receptors are immobilized on each well of a 96-well plate and then reacted, in an appropriate medium, with a test substance and the other receptors labeled with an enzyme such as horseradish peroxidase or alkaline phosphatase. After washing, a coloring reagent is added to the plate, and absorbance for each plate is measured. The receptor oligomerization can be detected by increased absorbance. Receptor oligomerization can also be detected using chemiluminescence by labeling the receptors with luciferase as an enzyme and developing the plate by a coloring reagent. Alternatively, radiolabeled or fluorescently labeled receptors can be used instead of the enzyme-labeled receptors to detect receptor oligomerization. In this case, the detection may be accomplished by the measurement of the remaining ionized radiation dose or the fluorescence intensity.

In another embodiment of the present invention, a gel filtration chromatography technique can be used. Two types of receptors and a test substance are reacted under appropriate conditions and then subjected to gel filtration chromatography. The receptor oligomerization can be detected by a shift of a retention time for peak absorbance.

A substance that inhibits the oligomerization of the receptor protein molecules or peptide fragments thereof may be screened as follows.

An oligomerization-inhibiting substance can be screened by detecting a change in the signal intensity in the presence of a test substance, together with a substance that promotes the oligomerization of the receptor protein molecules or peptide fragments thereof.

For example, the oligomerization-inhibiting substance can be detected by adding an oligomerization-promoting substance and a test substance to an aqueous solution comprising ¹²⁵I-labeled erythropoietin receptors or peptide fragments thereof as well as erythropoietin receptors or peptide fragments thereof attached to the surface of scintillant-containing yttrium silicate or polyvinyl toluene beads; and then measuring generated scintillation signals.

In one embodiment of the test kit according to the present invention, elements composing the kit may be packaged in a container(s), such as vial(s) or tube(s), separately or in combination or as a single unit. The container(s) may further be packaged as a single unit in a holder with several compartments. In this test kit, both of the receptor protein molecules or peptide fragments thereof and the oligomerization-promoting substance may be in lyophilized form. These elements may be reconstituted with a buffer when the test kit is used for the screening test.

This specification includes part or all of the contents as disclosed in the specification and/or drawings of Japanese Patent Application No. 10-102325 which is a priority document of the present application.

### BRIEF DESCRIPTION OF THE DRAWING

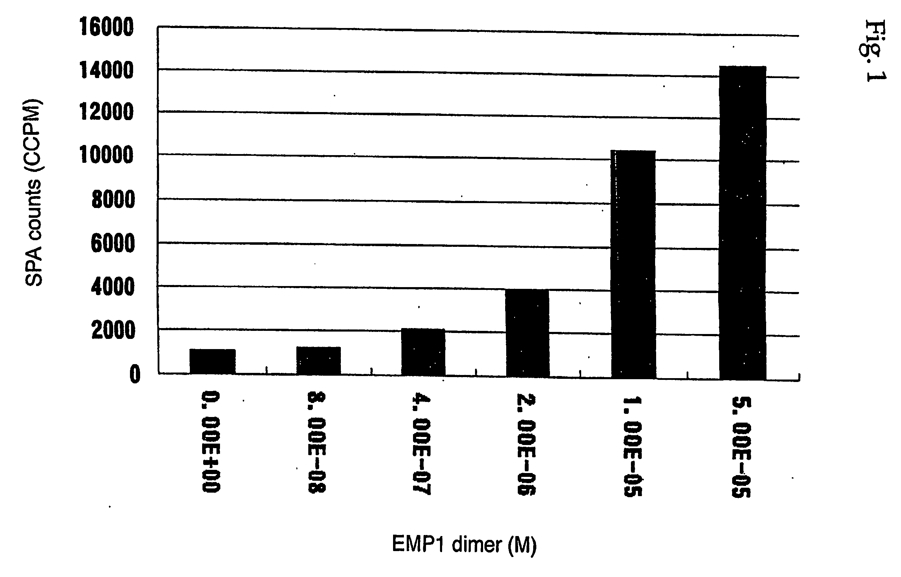

Figure 1 shows the results of EPO receptor dimer detection using SPA beads. The horizontal axis indicates a molar concentration of EMP1 dimer and the vertical axis indicates emission counts.

### BEST MODES FOR CARRYING OUT THE PRESENT INVENTION

The present invention will be further described in the following examples, which are provided for illustrative purposes only and are not intended to limit the scope of the invention.

### Example 1: Cloning of EPO receptor gene

1) Two pairs of PCR primers (i.e., 4 primers in total) were designed based on EPO receptor cDNA sequence. Namely, the following 5'-primer ERO-1 and 3'-primer ERO-2 were prepared for the first PCR amplification: To improve the accuracy of cloning, the following 5'-primer ERI-1 and 3'-primer ERI-2, which are internally adjacent to ERO-1 and ERO-2, respectively, were prepared for the second PCR amplification: The primers ERI-1 and ERI-2 include an EcoR I site and a Hind III site, respectively in order to simplify subsequent subcloning.
2) AS-E2 cell line highly expressing EPO receptors (about 1×10⁸ cells) was used to extract total RNA (1.23 mg) according to a modified AGPC method with phenol treatment and chloroform treatment. Oligo dT-latex beads were used to purify poly(A)+RNA (7.8 µg) from the whole total RNA. After confirming the mRNA purity by electrophoresis, 1 µg of poly(A)+RNA was subjected to reverse transcription reaction using an Amersham cDNA synthesis kit. Two microliters of the resulting reaction mixture (20 µl in total) was subjected to PCR using LA Taq (Takara LA PCR kit ver2). ERO-1 & ERO-2 and ERI-1 & ERI-2 were used as PCR primer pairs. The PCR reaction was carried out under the following conditions: 2 minutes at 94 °C, (20 seconds at 98 °C / 90 seconds at 60 °C/3 minutes at 72 °C)×30 cycles, and 10 minutes at 72 °C. A portion of this RT-PCR reaction mixture was electrophoresed in order to confirm the amplified fragment size.
3) The first strand cDNA synthesized from AS-E2 cell mRNA was used as a template and subjected to six separate PCR reactions using LA Taq (Takara), as described above. ERI-1 and ERI-2 were used as primers in these PCR reactions. Each of amplified PCR fragments was subcloned into a pBluescript II SK(+). One clone was selected for each PCR fragment to prepare a plasmid. The resulting six clone plasmids and PCR fragments (direct sequencing) were analyzed.
4) cDNA insert was excised with BamH I and Hind III from a EPO-R cDNA plasmid carrying two base substitutions, and then inserted into a BamH I/Hind III site of pUC 119 vector. This plasmid was digested with BssH II and Bal I to obtain a fragment including the vector (fragment 1). Meanwhile, a region between a BssH II site and a Bal I site was excised from a normal clone without any mutation in this region (fragment 2), which clone had been selected out of the six EPO-R cDNA clones prepared in 3) above. Fragments 1 and 2 were ligated to each other. The resulting clones were analyzed by sequencing, so as to isolate a clone into which fragment 2 had been correctly inserted (pUC 119-EPOR-L2). cDNA insert was excised from pUC 119-EPOR-L2 using BamH I and Hind III, and inserted into a BamH I/Hind III site of pBLSII SK(+) vector, thereby obtaining a clone (pEPOR-L2/SK).

### Example 2: Construction of expression system for soluble EPO receptors

1) To construct a human soluble EPO receptor gene, PCR was carried out using pEPOR-SK as a template and the following primers 1 and 2:
   A reaction mixture (50 µl) containing 10 mM Tris-HCl (pH 8.3), 50 mM KCI, 1.5 mM MgCl₂, 2.5U Ex. Taq (Takara Shuzo Co., Ltd.), 0.25 mM dNTP, 0.2 µM primers 1 and 2, and 180 ng pEPOR-SK was overlaid with 15 µl Chill Out 14 (MJ Research Inc.). PCR amplification was carried out in a 480-model thermal cycler (Perkin-Elmer) for 30 cycles under the following conditions: 1 minute at 96 °C, 1 minute at 60 °C, and 2 minutes at 72 °C, ending the amplification with an extra 10-minute holding at 72 °C. The resulting PCR products was purified using a PCR purification kit (Qiagen), precipitated in ethanol, and dissolved in 8 µl TE. After addition of 1 µl 10×Buffer H (Takara Shuzo Co., Ltd.), the purified PCR products were digested with EcoR I and BamH I (0.5 µl, 10U each) at 37 °C for 2 hours. The digested products were electrophoresed on 1 % agarose gel to cut out a band of approximately 700 bp. This band was extracted and purified using a gel extraction kit (Qiagen), precipitated in ethanol, and dissolved in 5 µl TE. Similarly, 1 µg pUC 19 (Takara Shuzo Co., Ltd.) was digested with EcoR I and BamH I, precipitated in ethanol, and dissolved in 10 µl TE. The band (2 µl) and pUC 19 (1 µl) thus obtained were mixed and reacted with 1 µl T4 DNA ligase (Life Technologies Oriental Inc.) in 4 µl distilled water and 2µl 5×T4 DNA ligase Buffer at 16 °C for 2 hours. The reaction mixture was then transformed into *E*. *coli* strain JM109 competent cells (Takara Shuzo Co., Ltd.) which had been melted on ice, and incubated for 30 minutes on ice. After additional incubation for 1 minute at 42 °C and then for 2.5 minutes on ice, the cells were further maintained at 37 °C in 500 µl SOC medium (Life Technologies Oriental Inc.) added thereto. The transformed cells (200 µl) were inoculated onto LBA-Amp plates and grown overnight at 37 °C. Six colonies were picked out of the resulting colonies, each of which was then inoculated into 3 ml LB-Amp medium and streaked on an LBA-Amp plate, followed by culturing at 37 °C. Subsequently, cells grown in each cell culture were suspended in distilled water and subjected to colony PCR under the conditions described above. The resulting PCR products were analyzed by agarose gel electrophoresis, confirming that each clone carries the inserted EPO receptor gene. *E*. *coli* cells were collected by centrifugation at 2,000 rpm for 15 minutes (Hitachi 05PR22) from each LB-Amp cell culture. A QIAprep-spin plasmid kit (Qiagen) was used to collect the plasmids in 100 µl TE. Each plasmid solution was analyzed by cycle sequencing in an ABI373A-model DNA sequencer (Perkin-Elmer) using a cycle sequencing kit (Perkin-Elmer), primers 1, 2, 3 and 4 and primer M13(-21) (Perkin-Elmer) or M13RV (Takara Shuzo Co., Ltd.). The plasmid solution prepared from a clone having the target sequence encoding the soluble EPO receptor was digested with EcoR I and BamH I under the conditions described above, followed by extraction and purification. The digested plasmid was ligated to pCHO1 which had been similarly digested and purified, and then transformed into *E*. *coli* strain JM109 competent cells under the conditions described above. Similarly, a plasmid solution was prepared and digested with EcoR I and BamH I, and then analyzed by agarose gel electrophoresis, thereby obtaining an expression plasmid pCHO/EpoR2 for the human soluble EPO receptor that carries the inserted target soluble EPO receptor gene.
2) Next, a CHO cell line expressing the human soluble EPO receptors was prepared.
   A solution of pCHO/EpoR2 plasmid (30 µl) was mixed with 4 µl 10×Buffer K (Takara Shuzo Co., Ltd.), 4 µl 1% BSA solution and 2 µl Pvu I and incubated overnight at 37 °C. After addition of 50 µl TE and 100 µl TE-saturated phenol, the solution was centrifuged at 14,000 rpm for 1 minute (MRX-150, Tomy Seiko Co., Ltd.) to recover an aqueous phase. This aqueous phase was mixed with 100 µl chloroform added thereto, and centrifuged at 14,000 rpm for 1 minute (MRX-150, Tomy Seiko Co., Ltd.) to recover an aqueous phase, followed by precipitation in ethanol. The precipitated products were dissolved in 20 µl sterile TE in order to measure its absorbance at 260 nm for calculation of its concentration. Ten microliters of Lipofect AMINE (Life Technologies Oriental Inc.) and 3 µg of Pvu I-digested plasmid solution were suspended in 0.3 ml α-MEM medium, followed by incubation at room temperature for 30 minutes. After washing with α-MEM, CHO cells grown to 70 % confluency in 25T flask (Becton Dickinson & Co.) were cultured in 2.4 ml α-MEM in the presence of the Lipofect AMINE-plasmid mixture at 37 °C under 5 % CO₂ for 8 hours. The supernatant was discarded and the cells were maintained overnight in 5 ml α-MEM supplemented with 10 % fetal calf serum (FCS). The supernatant was discarded again, and a cell culture was started in 5 ml α-MEM supplemented with 10% FCS (nucleic acid free, selective medium). The selective medium was changed every 2-4 days. After subculturing once in 75T flask, the cells were subjected to limiting dilution in five 96-1/2 well plates (Corning) such that the cells were diluted to 0.1 cells per well. Two weeks later, each well was observed by an inverted microscope to confirm proliferation of 89 clones. After washing with Dulbecco's PBS, the cells were collected by treating with a trypsin-EDTA solution. The collected cells were further grown in 24-well plates to confluency. The cell culture was continued for 4 days after changing the medium to obtain the culture supernatant. The soluble EPO receptors were detected by Western blot method using anti-FLAB M2 antibodies as primary antibodies, thereby obtaining a clone c165 expressing the soluble EPO receptors. The clone c165 was cultured in a selective medium containing 20 nM MTX, followed by limiting dilution, thereby obtaining 36 clones. The soluble EPO receptors in the culture supernatant were detected by Western blot method using anti-FLAG M2 antibodies or anti-EPO receptor antibodies (R&D systems) as primary antibodies, thereby obtaining a CHO cell line EpoR/CHO c165-19 expressing the soluble EPO receptors.

### Example 3: Purification of soluble EPO receptors

The culture supernatant of sEpoR-expressing CHO cells was filtered (pore size: 5 µm, Fuji Photo Film Co., Ltd.), and then passed through SARTOPURE PP filter (Sartorius) and SARTOBRAN P filter (pore size: 0.45+0.2 µm, Sartorius) to remove cell debris.

Bis-Tris HCl buffer was added to the resulting filtrate to 20 mM, which buffer had been prepared by dissolving Bis-Tris in water and adjusting to pH 6.0 with HCl. This filtrate was then applied at a flow rate of 4-5 ml/min to a Q Sepharose Fast Flow column (bed volume: 500 ml) equilibrated with 20 mM Bis-Tris HCl buffer (pH 6.0). This column was washed with 20 mM Bis-Tris HCl buffer (pH 6.0) to remove non-adsorbed protein, followed by elution with 20 mM Bis-Tris HCl buffer (pH 6.0) containing 0.5 M NaCl at a flow rate of 5-6 ml/min. Tris-HCl buffer was added to the resulting fraction to 50 mM, which buffer had been prepared by dissolving Tris in water and adjusting to pH 7.3 with HCl. This fraction was then applied at a flow rate of 1 ml/min to an anti-FLAG M2 affinity column (bed volume: 10ml, Eastman Kodak Co.) equilibrated with TBS solution (Takara Shuzo Co., Ltd.).

Proteins adsorbed to the anti-FLAG M2 affinity column were eluted with 0.1 M glycine HCl buffer (pH 3.5) and adjusted to neutral pH by adding about 1/10 volumes of 1 M Tris-HCl buffer (pH 8.0), followed by reversed-phase HPLC using a Vydac Protein C4 column (Vydac). That is, the proteins were adsorbed to a Vydac Protein C4 column equilibrated with a 0.1 % trifluoroacetic acid solution containing 20 % acetonitrile, and then eluted with a linear acetonitrile gradient up to 80% over 60 minutes. The elution using this HPLC technique resulted in a single elution peak, thereby obtaining purified sEpoR.

### Example 4: Biotinylation of soluble EPO receptors

The purified sEpoR from Example 3 was subjected to solvent replacement with 0.1 M sodium bicarbonate buffer (pH 8.6). Namely, sEpoR was dissolved in 0.1 M sodium bicarbonate buffer (pH 8.6) after drying in a centrifugal concentrator under reduced pressure, or was applied to a Fast Desalting column (Pharmacia) equilibrated with 0.1 M sodium bicarbonate buffer (pH 8.6).

A biotinylating reagent (Amersham) was added to the proteins to be labeled in an amount of 40 µl per mg protein, and reacted at room temperature for one hour while stirring. The reaction mixture was applied to a Bio-Gel P-6 column (Bio-Rad Laboratories) equilibrated with PBS solution containing 0.02 % Tween 20 to remove unreacted reagents, thereby obtaining a protein fraction containing biotinylated sEpoR.

### Example 5: Labeling of soluble EPO receptors with ¹²⁵I

The purified sEpoR from Example 3 was subjected to solvent replacement with 0.1 M sodium bicarbonate buffer (pH 8.6). Namely, sEpoR was dissolved in 0.1 M sodium bicarbonate buffer (pH 8.6) after drying in a centrifugal concentrator under reduced pressure, or was applied to a Fast Desalting column (Pharmacia) equilibrated with 0.1 M sodium bicarbonate buffer (pH 8.6).

A vial containing ¹²⁵I-Bolton and Hunter reagent (Amersham) was exposed to a nitrogen gas stream to evaporate the solvent contained therein. Fifteen microliters of sEpoR solution (ca 1 mg/ml) was added to this vial and reacted with the reagent on ice for 30 minutes while stirring every 5 minutes. The reaction continued for additional 5 minutes on ice after addition of 500 µl 0.1 M sodium bicarbonate buffer (pH 8.6) containing 0.2 M glycine.

The reaction mixture was applied to a Sephadex G-25 column (Pharmacia) equilibrated with 0.1 M phosphate buffer (pH 7.2) containing 0.25 % gelatin to remove unreacted reagents, thereby obtaining a protein fraction containing ¹²⁵I-labeled sEpoR.

### Example 6: Detection of EPO receptor dimerization using SPA beads

50 µg streptavidin-attached SPA beads (Amersham), biotinylated sEpoR and about 150,000 cpm ¹²⁵I-labeled sEpoR were dissolved in 100 µl test buffer (Dulbecco's PBS(-) containing 0.1 % BSA and 5 mM EDTA) to prepare a test solution. Meanwhile, EPO receptor-binding peptide dimers were prepared by asking Peptide Institute, Inc. to synthesize them as described in Science, 273:458-463, 1996; Blood, 88(10):542a, 1996; Science, 276:1696-1699, 1997. The EPO receptor-binding peptide dimers were mixed and reacted with the test solution at room temperature for 12 hours, followed by detection using Microbeta (Pharmacia).

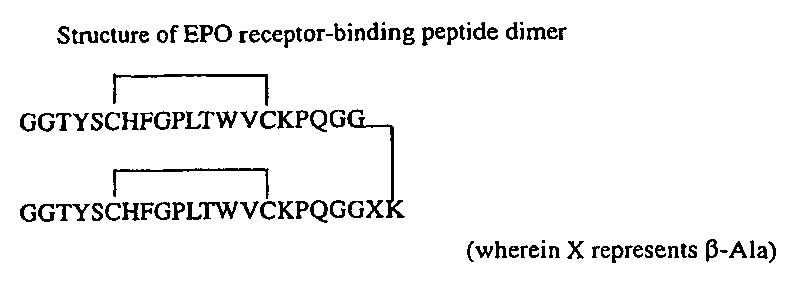

Signals whose intensity depends on the concentration of the EMP 1 dimer were generated from the SPA beads, resulting in the detection of EPO receptor dimerization (Figure 1).

### INDUSTRIAL APPLICABILITY

The present invention provides a method for screening substitutes for physiologically active substances, which uses receptor multimerization as an indicator.

## Claims

1. A method for screening substances that promote oligomerization of receptor protein molecules or peptide fragments thereof, which comprises determining whether the receptor protein molecules or peptide fragments thereof are oligomerized when contacted with a test substance.

2. The method of claim 1, wherein the receptor protein molecules or peptide fragments thereof are contacted with the test substance in a cell-free environment.

3. The method of claim 1 or 2, which comprises measuring signals generated by the oligomerization of the receptor protein molecules or peptide fragments thereof in order to determine whether the receptor protein molecules or peptide fragments thereof are oligomerized.

4. The method of claim 3, wherein the signal is selected from the group consisting of scintillation signal, chemiluminescence signal, fluorescence signal, absorption signal, ionizing radiation signal, nuclear magnetic resonance signal and surface plasmon resonance signal.

5. The method of claim 3, wherein at least one of the receptor protein molecules or peptide fragments thereof is labeled.

6. The method of claim 5, wherein the label is selected from the group consisting of radioisotope, fluorophore, enzyme, biotin, avidin, scintillant, and combinations thereof.

7. The method of claim 6, wherein at least one of the receptor protein molecules or peptide fragments thereof is radiolabeled, while at least one of the others is attached to the surface of beads containing scintillant.

8. The method of claim 7, which comprises:
adding the test substance to an aqueous solution comprising the radiolabeled receptors or peptide fragments thereof as well as the receptors or peptide fragments thereof attached to scintillant-containing beads; and
measuring generated scintillation signals to determine whether the receptors or peptide fragments thereof are oligomerized.

9. The method of claim 1, wherein the oligomer of the receptor protein molecules or peptide fragments thereof is formed in a solution.

10. The method of claim 1, wherein the oligomer of the receptor protein molecules or peptide fragments thereof is formed on a solid surface.

11. The method of any one of claims 1 to 10, wherein the receptor is a cytokine receptor.

12. The method of claim 11, wherein the cytokine receptor is selected from the group consisting of hematopoietic factor receptor, lymphokine receptor, growth factor receptor and differentiation inhibitory factor receptor.

13. The method of claim 11, wherein the cytokine receptor is selected from the group consisting of erythropoietin (EPO) receptor, thrombopoietin (TPO) receptor, granulocyte colony-stimulating factor (G-CSF) receptor, macrophage colony-stimulating factor (M-CSF) receptor, granulocyte-macrophage colony-stimulating factor (GM-CSF) receptor, tumor necrosis factor (TNF) receptor, interleukin-1 (IL-1) receptor, interleukin-2 (IL-2) receptor, interleukin-3 (IL-3) receptor, interleukin-4 (IL-4) receptor, interleukin-5 (IL-5) receptor, interleukin-6 (IL-6) receptor, interleukin-7 (IL-7) receptor, interleukin-9 (IL-9) receptor, interleukin-10 (IL-10) receptor, interleukin-11 (IL-11) receptor, interleukin-12 (IL-12) receptor, interleukin-13 (IL-13) receptor, interleukin-15 (IL-15) receptor, interferon-α (IFN-α) receptor, interferon-β (IFN-β) receptor, interferon-γ (IFN-γ) receptor, growth hormone (GH) receptor, insulin receptor, stem cell factor (SCF) receptor, vascular endothelial growth factor (VEGF) receptor, epidermal growth factor (EGF) receptor, nerve growth factor (NGF) receptor, fibroblast growth factor (FGF) receptor, platelet-derived growth factor (PDGF) receptor, transforming growth factor-β (TGF-β) receptor, leukemia inhibitory factor (LIF) receptor, ciliary neurotrophic factor (CNTF) receptor, oncostatin M (OSM) receptor and Notch-like receptor.

14. The method of claim 11, wherein the receptor protein molecule is a cytokine receptor subunit.

15. The method of any one of claims 1 to 14, wherein the peptide fragment of the receptor protein molecule comprises a soluble region of the receptor.

16. The method of claim 15, wherein the peptide fragment of the receptor protein molecule comprises an extracellular region of the receptor.

17. The method of claim 15, wherein the peptide fragment of the receptor protein molecule comprises at least a ligand-binding site of the receptor.

18. The method of any one of claims 1 to 17, wherein the oligomer is a homooligomer.

19. The method of any one of claims 1 to 17, wherein the oligomer is a heterooligomer.

20. The method of any one of claims 1 to 19, wherein the oligomer is a dimer.

21. The method of any one of claims 1 to 20, wherein the substance to be screened is a novel substitute for a known physiologically active substance.

22. A method for screening substances that promote dimerization of erythropoietin receptor protein molecules or peptide fragments thereof, which comprises:
adding a test substance to an aqueous solution comprising ¹²⁵I-labeled erythropoietin receptors or peptide fragments thereof as well as erythropoietin receptors or peptide fragments thereof attached to the surface of scintillant-containing yttrium silicate or polyvinyl toluene beads; and
measuring generated scintillation signals to determine whether the erythropoietin receptors or peptide fragments thereof are dimerized.

23. A method for screening substances that inhibit oligomerization of receptor protein molecules or peptide fragments thereof, which comprises determining whether oligomerization of the receptor protein molecules or peptide fragments thereof in the presence of an oligomerization-promoting substance is inhibited by contact with a test substance.

24. The method of claim 23, wherein the oligomerization-promoting substance has a physiological activity.

25. The method of claim 24, wherein the substance that inhibits the oligomerization of receptor protein molecules or peptide fragments thereof has the ability to inhibit the physiological activity of the oligomerization-promoting substance.

26. The method of claim 23, wherein the oligomerization-promoting substance is erythropoietin, and the receptor is an erythropoietin receptor.

27. A test kit for screening substances that inhibit oligomerization of receptor protein molecules or peptide fragments thereof, which comprises an oligomerization-promoting substance, the receptor protein molecules or peptide fragments thereof, and a buffer.

28. The test kit of claim 27, wherein the oligomerization-promoting substance is erythropoietin, the receptor protein molecule or peptide fragment thereof is an erythropoietin receptor protein molecule or peptide fragment thereof, and the buffer is phosphate buffered saline.

29. Use of receptor protein molecules or peptide fragments thereof for determining whether a test substance promotes oligomerization of the receptor protein molecules or peptide fragments thereof.

## Revendications

1. Méthode pour le criblage de substances qui favorisent l'oligomérisation des molécules des protéines réceptrices ou de leurs fragments de peptide, qui comprend la détermination du fait que les molécules des protéines réceptrices ou leurs fragments de peptide sont oligomérisés quand ils sont mis en contact avec une substance test ou non.

2. Méthode de la revendication 1, où les molécules des protéines réceptrices ou leurs fragments de peptide sont mis en contact avec la substance test dans un environnement sans cellule.

3. Méthode de la revendication 1 ou 2, qui comprend la mesure des signaux générés par l'oligomérisation des molécules des protéines réceptrices et leurs fragments de peptide afin de déterminer si les molécules des protéines réceptrices et leurs fragments de peptide sont oligomérisés.

4. Méthode de la revendication 3, où le signal est sélectionné dans le groupe consistant en signal de scintillation, signal de chimioluminescence, signal de fluorescence, signal d'absorption, signal d'un rayonnement ionisant, signal de résonance magnétique nucléaire et signal de résonance de plasmon de surface.

5. Méthode de la revendication 3, où au moins l'une des molécules des protéines réceptrices ou leurs fragments de peptide est marquée.

6. Méthode de la revendication 5, où le marqueur est sélectionné dans le groupe consistant en radioisotope, fluorophore, enzyme, biotine, avidine, agent scintillant et leurs combinaisons.

7. Méthode de la revendication 6, où au moins l'un des molécules des protéines réceptrices ou leurs fragments de peptide est radiomarquée, alors qu'au moins l'un des autres est attaché à la surface de perles contenant un agent scintillant.

8. Méthode de la revendication 7, qui comprend:
l'addition de la substance test à une solution aqueuse comprenant les récepteurs radiomarqués ou leurs fragments de peptide ainsi que les récepteurs ou fragments de peptide attachés à des perles contenant un agent scintillant; et
la mesure des signaux générés de scintillation pour déterminer si les récepteurs ou leurs fragments de peptide sont oligomérisés.

9. Méthode de la revendication 1, où l'oligomère ou les molécules des protéines réceptrices ou leurs fragments de peptide est formé dans une solution.

10. Méthode de la revendication 1, où l'oligomère des molécules des protéines réceptrices ou leurs fragments de peptide est formé sur une surface solide.

11. Méthode de l'une quelconque des revendications 1 à 10, où le récepteur est un récepteur de cytokine.

12. Méthode de la revendication 11, où le récepteur de cytokine est sélectionné dans le groupe consistant en récepteur du facteur hématopoïétique, récepteur de lymphokine, récepteur du facteur de croissance et récepteur du facteur inhibiteur de la différenciation.

13. Méthode de la revendication 11, où le récepteur de cytokine est sélectionné dans le groupe consistant en récepteur d'érythropoïétine (EPO), récepteur de thrombopoiétine (TPO), récepteur du facteur de stimulation de colonies de granulocytes (G-CSF), récepteur du facteur de stimulation des colonies de macrophages (M-CSF), récepteur du facteur de simulation de colonies de macrophages-granulocytes (GM-CSF), récepteur du facteur de nécrose de la tumeur (TNF), récepteur d'interleukine-1 (IL-1), récepteur d'interleukine-2 (IL-2), récepteur d'interleukine-3 (IL-3), récepteur d'interleukine-4 (IL-4), récepteur d'interleukine-5 (IL-5), récepteur d'interleukine-6 (IL-6), récepteur d'interleukine-7 (IL-7), récepteur d'interleukine-8 (IL-8), récepteur d'interleukine-9 (IL-9), récepteur d'interleukine-10 (IL-10), récepteur d'interleukine-11 (IL-11), récepteur d'interleukine-12 (IL-12), récepteur d'interleukine-13 (IL-13), récepteur d'interleukine-14 (IL-14), récepteur d'interleukine-15 (IL-15), récepteur de l'interféron-α (IFN-α), récepteur de l'interféron-β (IFN-β), récepteur de l'interféron-γ (IFN-γ), récepteur du facteur de l'hormone de croissance (GH), récepteur d'insuline, récepteur du facteur des cellules souches (SCF), récepteur du facteur de croissance endothéliale vasculaire (VEGF), récepteur de facteur de croissance épidermique (EGF), récepteur du facteur de croissance des nerfs (NGF), récepteur du facteur de croissance des fibroblastes (FGF), récepteur du facteur de croissance dérivé des plaquettes (PDGF), récepteur du facteur-β de croissance transformante (TGF-β), récepteur du facteur inhibiteur de la leucémie (LIF), récepteur du facteur neurotrophe ciliaire (CNTF), récepteur de l'oncostatine M (OSM) et récepteur comme Notch.

14. Méthode de la revendication 11, où la molécule de la protéine réceptrice est une sous-unité réceptrice de cytokine.

15. Méthode de l'une quelconque des revendications 1 à 14, où le fragment de peptide de la molécule de la protéine réceptrice forme une région soluble du récepteur.

16. Méthode de la revendication 15, où le fragment de peptide de la molécule de la protéine réceptrice forme une région extra-cellulaire du récepteur.

17. Méthode de la revendication 15, où le fragment de peptide de la molécule de la protéine réceptrice forme au moins un site de liaison du ligand du récepteur.

18. Méthode de l'une quelconque des revendications 1 à 17, où l'oligomère est un homooligomère.

19. Méthode de l'une quelconque des revendications 1 à 17, où l'oligomère est un hétérooligomère.

20. Méthode de l'une quelconque des revendications 1 à 19, où l'oligomère est un dimère.

21. Méthode de l'une quelconque des revendications 1 à 20, où la substance à cribler est un nouveau substitut d'une substance physiologiquement active connue.

22. Méthode pour le criblage des substances qui favorisent la dimérisation des molécules des protéines réceptrices d'érythropoïétine ou leurs fragments de peptide, qui comprend:
l'addition d'une substance test à une solution aqueuse comprenant des récepteurs d'érythropoïétine marqués ¹²⁵I ou leurs fragments de peptide ainsi que des récepteurs de l'érythropoïétine ou leurs fragments de peptide attachés à la surface de silicate d'yttrium contenant un agent scintillant ou de perles de polyvinyl toluène; et
la mesure des signaux générés de scintillation pour déterminer si les récepteurs d'érythropoïétine ou leurs fragments de peptide sont dimérisés.

23. Méthode pour le criblage de substances qui inhibent l'oligomérisation des molécules des protéines réceptrices ou leurs fragments de peptide, qui comprend la détermination du fait que l'oligomérisation des molécules des protéines réceptrices ou leurs fragments de peptide en présence d'une substance favorisant l'oligomérisation est inhibée par contact avec une substance test ou non.

24. Méthode de la revendication 23, où la substance favorisant l'oligomérisation a une activité physiologique.

25. Méthode de la revendication 24, où la substance qui inhibe l'oligomérisation des molécules des protéines réceptrices ou leurs fragments de peptide a l'aptitude d'inhiber l'activité physiologique de la substance favorisant l'oligomérisation.

26. Méthode de la revendication 23, où la substance favorisant l'oligomérisation est l'érythropoïétine et le récepteur est un récepteur d'érythropoïétine.

27. Kit de test pour cribler des substances qui inhibent l'oligomérisation des molécules des protéines réceptrices ou leurs fragments de peptide, qui comprend une substance favorisant l'oligomérisation, les molécules des protéines réceptrices ou leurs fragments de peptide et un tampon.

28. Kit de test de la revendication 27, où la substance favorisant l'oligomérisation est l'érythropoïétine, la molécule de la protéine réceptrice ou son fragment de peptide est une molécule d'une protéine réceptrice d'érythropoïétine ou son fragment de peptide et le tampon est une solution saline tamponnée phosphate.

29. Utilisation de molécules de protéines réceptrices ou leurs fragments de peptide pour déterminer si une substance test favorise l'oligomérisation des molécules des protéines réceptrices ou leurs fragments de peptide ou non.

## Patentansprüche

1. Verfahren zum Screenen auf Substanzen, welche die Oligomerisation von Rezeptorproteinmolekülen oder Peptidfragmenten davon fördern, umfassend das Bestimmen, ob die Rezeptorproteinmoleküle oder Peptidfragmente davon oligomerisiert werden, wenn sie mit einer Testsubstanz in Kontakt kommen.

2. Verfahren nach Anspruch 1, worin die Rezeptorproteinmoleküle oder Peptidfragmente davon mit der Testsubstanz in einer zellfreien Umgebung kontaktiert werden.

3. Verfahren nach Anspruch 1 oder 2, umfassend das Messen von Signalen, die durch die Oligomerisation der Rezeptorproteinmoleküle oder Peptidfragmente davon erzeugt werden, um zu bestimmen, ob die Rezeptorproteinmoleküle oder Peptidfragmente davon oligomerisiert werden.

4. Verfahren nach Anspruch 3, worin das Signal aus der aus Szintillations-, Chemolumineszenz-, Fluoreszenz-, Absorptions-, ionisierenden Strahlungs-, magnetischen Kernresonanz- und Oberflächenplasmonresonanz-Signalen bestehenden Gruppe ausgewählt wird.

5. Verfahren nach Anspruch 3, worin zumindest eines der Rezeptorproteinmoleküle oder Peptidfragmente davon markiert wird.

6. Verfahren nach Anspruch 5, worin die Markierung aus der aus Radioisotop, Fluorophor, Enzym, Biotin, Avidin, Szintillationssubstanz und Kombinationen davon bestehenden Gruppe ausgewählt wird.

7. Verfahren nach Anspruch 6, worin zumindest eines der Rezeptorproteinmoleküle oder Peptidfragmente davon radioaktiv markiert wird, während zumindest eines der anderen an die Oberfläche von Szintillationssubstanz-enthaltenden Perlen gebunden wird.

8. Verfahren nach Anspruch 7, umfassend:
das Zusetzen der Testsubstanz zu einer wässrigen Lösung, welche die radioaktiv markierten Rezeptoren oder Peptidfragmente davon sowie die an Szintillationssubstanz-hältige Perlen gebundenen Rezeptoren oder Peptidfragmente davon umfasst; und
das Messen von erzeugten Szintillationssignalen, um zu bestimmen, ob die Rezeptoren oder Peptidfragmente davon oligomerisiert werden.

9. Verfahren nach Anspruch 1, worin das Oligomer der Rezeptorproteinmoleküle oder Peptidfragmente davon in einer Lösung gebildet wird.

10. Verfahren nach Anspruch 1, worin das Oligomer der Rezeptorproteinmoleküle oder Peptidfragmente davon an einer festen Oberfläche gebildet wird.

11. Verfahren nach einem der Ansprüche 1 bis 10, worin der Rezeptor ein Cytokinrezeptor ist.

12. Verfahren nach Anspruch 11, worin der Cytokinrezeptor aus der aus Blutbildungsfaktorrezeptor, Lymphokinrezeptor, Wachstumsfaktorrezeptor und Differenzierungshemmfaktor-Rezeptor bestehenden Gruppe ausgewählt wird.

13. Verfahren nach Anspruch 11, worin der Cytokinrezeptor aus der aus Erythropoietin- (EPO-) Rezeptor, Thrombopoietin- (TPO-) Rezeptor, Granulozytenkoloniestimulierendem Faktor- (G-CSF-) Rezeptor, Makrophagenkolonie-stimulierendem Faktor- (M-CSF-) Rezeptor, Granulozyten-Makrophagenkolonie-stimulierendem Faktor- (GM-CSF-) Rezeptor, Tumornekrosefaktor- (TNF-) Rezeptor, Interleukin-1- (IL-1-) Rezeptor, Interleukin-2- (IL-2-) Rezeptor, Interleukin-3- (IL-3-) Rezeptor, Interleukin-4- (IL-4-) Rezeptor, Interleukin-5- (IL-5-) Rezeptor, Interleukin-6- (IL-6-) Rezeptor, Interleukin-7- (IL-7-) Rezeptor, Interleukin-9- (IL-9-) Rezeptor, Interleukin-10- (IL-10-) Rezeptor, Interleukin-11- (IL-11-) Rezeptor, Interleukin-12- (IL-12-) Rezeptor, Interleukin-13- (IL-13-) Rezeptor, Interleukin-15- (IL-15-) Rezeptor, Interferon-α- (IFN-α-) Rezeptor, Interferon-β- (IFN-β-) Rezeptor, Interferon-γ- (IFN-γ-) Rezeptor, Wachstumshormon- (GH-) Rezeptor, Insulinrezeptor, Stammzellenfaktor- (SCF-) Rezeptor, Gefäßendothelwachstumsfaktor- (VEGF-) Rezeptor, Epidermiswachstumsfaktor-(EGF-) Rezeptor, Nervenwachstumsfaktor- (NGF-) Rezeptor, Fibroblastenwachstumsfaktor- (FGF-) Rezeptor, Blutplättchen-abgeleitetem Wachstumsfaktor- (PDGF-) Rezeptor, transformierendem Wachstumsfaktor-β- (TGF-β-) Rezeptor, Leukämiehemmfaktor- (LIF-) Rezeptor, ziliarneurotrophischem Faktor- (CNTF-) Rezeptor, Oncostatin-M- (OSM-) Rezeptor und kerbenähnlichem Rezeptor bestehenden Gruppe ausgewählt wird.

14. Verfahren nach Anspruch 11, worin das Rezeptorproteinmolekül eine Cytokinrezeptor-Untereinheit ist.

15. Verfahren nach einem der Ansprüche 1 bis 14, worin das Peptidfragment des Rezeptorproteinmoleküls eine lösliche Region des Rezeptors umfasst.

16. Verfahren nach Anspruch 15, worin das Peptidfragment des Rezeptorproteinmoleküls eine extrazelluläre Region des Rezeptors umfasst.

17. Verfahren nach Anspruch 15, worin das Peptidfragment des Rezeptorproteinmoleküls zumindest eine Ligandenbindungsstelle des Rezeptors umfasst.

18. Verfahren nach einem der Ansprüche 1 bis 17, worin das Oligomer ein Homooligomer ist.

19. Verfahren nach einem der Ansprüche 1 bis 17, worin das Oligomer ein Heterooligomer ist.

20. Verfahren nach einem der Ansprüche 1 bis 19, worin das Oligomer ein Dimer ist.

21. Verfahren nach einem der Ansprüche 1 bis 20, worin die zu screenende Substanz ein neuer Ersatzstoff für eine bekannte physiologisch aktive Substanz ist.

22. Verfahren zum Screenen auf Substanzen, welche die Dimerisation von Erythropoietin-Rezeptorproteinmolekülen oder Peptidfragmenten davon fördern, umfassend:
das Zusetzen einer Testsubstanz zu einer wässrigen Lösung, die ¹²⁵I-markierte Erythropoietin-Rezeptoren oder Peptidfragmente davon sowie an die Oberfläche von Szintillationssubstanz-hältigen Yttriumsilicat- oder Polyvinyltoluol-Perlen gebundene Erythropoietin-Rezeptoren oder Peptidfragmente davon umfasst; und
das Messen erzeugter Szintillationssignale, um zu bestimmen, ob die Erythropoietin-Rezeptoren oder Peptidfragmente davon dimerisiert werden.

23. Verfahren zum Screenen auf Substanzen, welche die Oligomerisation von Rezeptorproteinmolekülen oder Peptidfragmenten davon hemmen, umfassend das Bestimmen, ob Oligomerisation der Rezeptorproteinmoleküle oder Peptidfragmente davon in Gegenwart einer die Oligomerisation fördernden Substanz durch Kontakt mit einer Testsubstanz gehemmt wird.

24. Verfahren nach Anspruch 23, worin die die Oligomerisation fördernde Substanz physiologische Aktivität aufweist.

25. Verfahren nach Anspruch 24, worin die Substanz, welche die Oligomerisation von Rezeptorproteinmolekülen oder Peptidfragmenten davon hemmt, die Fähigkeit besitzt, die physiologische Aktivität der die Oligomerisation fördernden Substanz zu hemmen.

26. Verfahren nach Anspruch 23, worin die die Oligomerisation fördernde Substanz Erythropoietin ist und der Rezeptor ein Erythropoietin-Rezeptor ist.

27. Testset zum Screenen auf Substanzen, welche die Oligomerisation von Rezeptorproteinmolekülen oder Peptidfragmenten davon hemmen, umfassend eine die Oligomerisation fördernde Substanz, die Rezeptorproteinmoleküle oder Peptidfragmente davon und einen Puffer.

28. Testset nach Anspruch 27, worin die die Oligomerisation fördernde Substanz Erythropoietin, das Rezeptorproteinmolekül oder das Peptidfragment davon ein Erythropoietin-Rezeptorproteinmolekül oder ein Peptidfragment davon und der Puffer phosphatgepufferte Salzlösung ist.

29. Verwendung von Rezeptorproteinmolekülen oder Peptidfragmenten davon zur Bestimmung, ob eine Testsubstanz die Oligomerisation der Rezeptorproteinmoleküle oder Peptidfragmente davon fördert.
